# EUROPÄISCHE PATENTSCHRIFT

(11) **EP 0 847 381 B1**
(45) Veröffentlichungstag und Bekanntmachung des Hinweises auf die Patenterteilung: **21.06.2000**
(21) Anmeldenummer: 96930051.6
(22) Anmeldetag: 19.08.1996
(51) Int. Cl.: C07C 227/18

(54) **VERFAHREN ZUR HERSTELLUNG VON N-CARBOXYMETHYLEN-4-CHLOR-ANTHRANILSÄURE, SOWIE DEREN DIALKYLESTERN**
PROCESS FOR PREPARING N-CARBOXYMETHYLENE-4-CHLORO-ANTHRANILIC ACID AND ITS DIALKYL ESTERS
PROCEDE DE PREPARATION D'ACIDE N-CARBOXYMETHYLENE-4-CHLORO-ANTHRANILIQUE ET DE SES ESTERS DE DIALKYLE

(30) Priorität: 31.08.1995 DE 19532054
(43) Veröffentlichungstag der Anmeldung: 17.06.1998
(73) Patentinhaber: Clariant GmbH, 65929 Frankfurt am Main (DE)
(72) Erfinder: PAPENFUHS, Theodor, D-60433 Frankfurt am Main (DE); PFIRMANN, Ralf, D-64347 Griesheim (DE); KRAUSE, Stefan, D-65929 Frankfurt (DE); NEUMANN-GRIMM, Doris, D-60388 Frankfurt am Main (DE)
(86) Internationale Anmeldenummer: EP9603630
(87) Internationale Veröffentlichungsnummer: WO9708129

(56) Entgegenhaltungen:
- EP-A- 0 071 935
- DE-C- 142 507
- JOURNAL OF HETEROCYCLIC CHEMISTRY, Bd. 24, 1987, PROVO US, Seiten 811-815, XP000615417 P.C. UNANGST ET AL.: "Synthesis of Novel 1-phenyl-1h-indole-2-carboxylic acids.I." in der Anmeldung erwähnt
- CHEMICAL ABSTRACTS, vol. 103, no. 1, 1985 Columbus, Ohio, US; abstract no. 5969c, J. ROMANOWSKI ET AL: "2,6-Dichloro-3-nitrobenzoic acid as a synthon for nucleophilic reactions." Seite 537; XP002021863 & POL. J. CHEM., Bd. 58, Nr. 1-2-3, 1984, Seiten 263-268,

## Beschreibung

Die Erfindung betrifft ein Verfahren zur Herstellung von N-Carboxymethylen-4-chlor-anthranilsäure, sowie deren Dialkylestern.

Diese Verbindungen sind wichtige Ausgangsstoffe für die Synthese von Aldose-Reduktase Hemmern.

N-Carboxymethylen-4-chlor-anthranilsäure kann nach J. Med. Chem. 1991, 34, 1283 durch Umsetzung von 4-Chloranthranilsäure mit Chloressigsäure hergestellt werden. Nachteilig hierbei ist die Verwendung der giftigen Chloressigsäure in der Substitutionsreaktion. In J. Prakt. Chem. 1929, 120, 64 ist die Reaktion von 4-Chloranthranilsäure mit KCN und Formaldehyd und anschließender Umsetzung des Zwischenproduktes mit wäßriger KOH beschrieben.
N-Carbmethoxymethylen-4-chlor-anthranilsäuremethylester ist durch Veresterung der N-Carboxymethylen-4-chlor-anthranilsäure mit Methanol/Schwefelsäure (J. Heterocyc. Chem. 1987, 24,811, J. Med. Chem. 1991, 34, 1283) mit Methanol, HCI (J. Prakt. Chem. 1929, 120, 64) zugänglich. Für die Veresterung sind ein extrem hoher Schwefelsäureüberschuß und sehr lange Reaktionszeiten nötig.

In der EP 71 935 sind folgende Reaktionssequenzen beschrieben

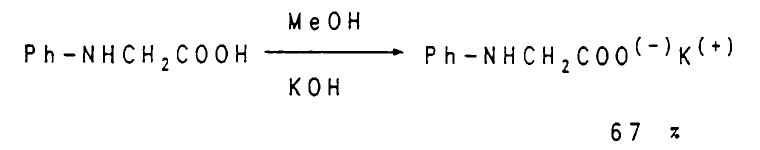

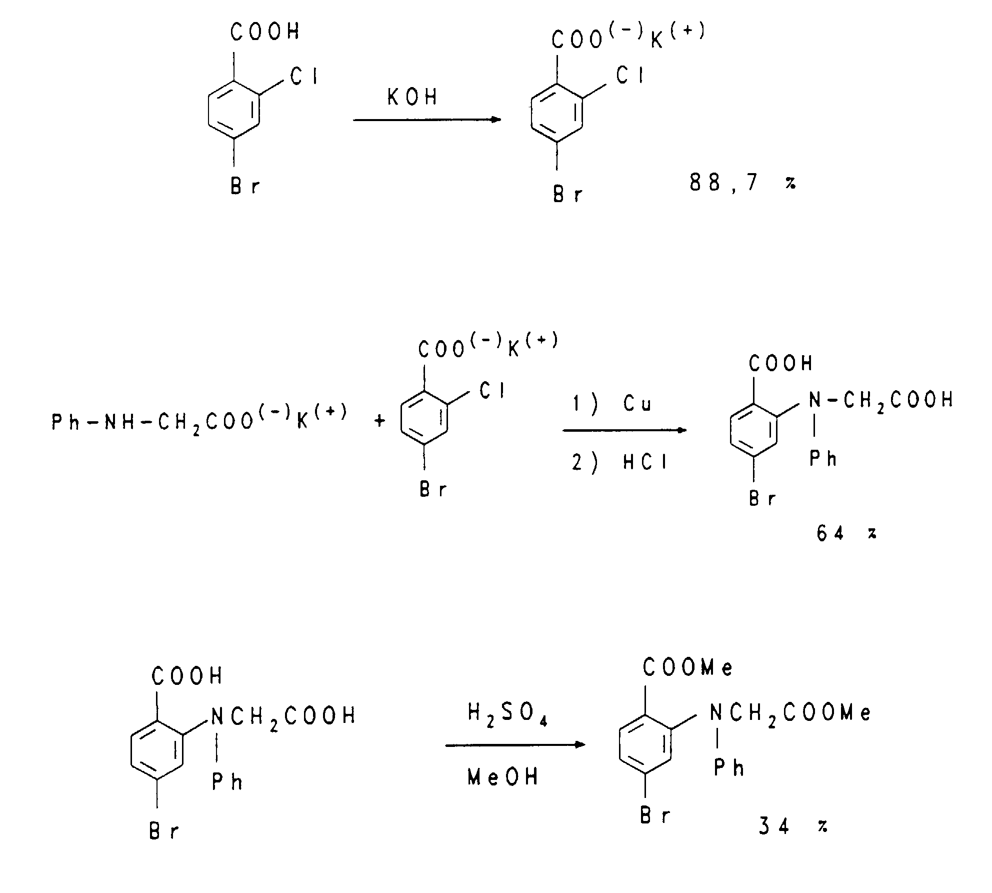

Die Gesamtausbeute des Diesters beträgt also nach dieser 4-stufigen Synthese (bezogen auf 3-Brom-6-chlor-benzoesäure) lediglich 19,3 %.

Es bestand somit ein Bedarf nach einem Verfahren, das die beschriebenen Nachteile vermeidet und N-Carboxymethylen-4-chlor-anthranilsäure sowie deren Dialkylester in hoher Ausbeute und Reinheit zugänglich macht.

Diese Aufgabe wird gelöst durch ein Verfahren zur Herstellung von N-Carboxymethylen-4-chlor-anthranilsäure und deren Dialkylestern, dadurch gekennzeichnet, daß man 2,4-Dichlorbenzoesäure in Gegenwart einer Base, eines Lösungsmittels und eines Katalysators aus Kupfer oder Kupferverbindungen mit Glycin zu N-Carboxymethylen-4-chlor-anthranilsäure umsetzt und gegebenenfalls diese in Gegenwart einer Base und eines Lösungsmittels mit einem Dialkylsulfat, Dialkylcarbonat oder Alkylhalogenid zum Dialkylester umsetzt.

Es hat sich bewährt, zur Herstellung von N-Carboxymethylen-4-chloranthranilsäure 0,5 bis 10 Mol, vorteilhaft 1 bis 5 Mol, bevorzugt 1 bis 2 Mol Glycin pro Mol Dichlorbenzoesäure einzusetzen. Als Lösungsmittel können dipolare aprotische Lösungsmittel, z.B. N,N-Dimethylacetamid, N,N-Diethylacetamid, N,N-Dimethylformamid, N-Methylpyrrolidon, Dimethylsulfoxid, Dimethylsulfon, Diphenylsulfon, Sulfolan, Tetramethylharnstoff, Tetra-n-butylharnstoff, 1,3-Dimethylindazolidin-2-on, Wasser oder protische organische Lösungsmittel wie primäre oder sekundäre Alkohole, besonders hochsiedende wie Ethylenglykol oder Glycerin, aliphatische Amine oder Mischungen der angegebenen Lösungsmittel dienen. Als günstig haben sich Dimethylacetamid, N-Methylpyrrolidon oder Wasser erwiesen. Katalytisch wirken Kupfer oder Kupferverbindungen, z.B. Cu oder basisches Kupfercarbonat, Kupferiodid oder Mischungen derselben. Man setzt 0,1 bis 20 mol-% des Katalysators ein, vorteilhaft 0,2 bis 10 mol-%, bevorzugt 0,5 bis 5 mol-%.

Als Base können Alkali- oder Erdalkalicarbonate, -hydrogencarbonate, -hydroxide, basische Oxide, -phosphate, -hydrogenphosphate oder -dihydrogenphosphate eingesetzt werden, auch Mischungen derselben. Vorteilhaft ist die Verwendung von Kaliumcarbonat, gegebenenfalls auch in Kombination mit einem Alkalimetallhydroxid, bevorzugt NaOH oder KOH. Die Basenmenge beträgt 1 bis 10 Mol pro Mol Dichlorbenzoesäure, vorteilhaft 1,2 bis 6, bevorzugt 1,5 bis 4 Mol pro Mol Dichlorbenzoesäure.

Die Reaktionstemperaturen hält man zwischen 40 und 200°C, vorteilhaft zwischen 50 und 160°C, bevorzugt 60 bis 150°C.

Die Reaktionszeiten betragen zwischen 30 min und 20 h, vorteilhaft zwischen 3 und 10 h, bevorzugt 2 bis 8 h.

Die Konzentration der 2,4-Dichlorbenzoesäure im Lösungsmittel hält man zwischen 1 und 90 Gew.-%, vorteilhaft zwischen 3 und 80 Gew.-%, bevorzugt zwischen 5 bis 70 Gew.-%.

Es wurde gefunden, daß die Veresterung beider Carboxylgruppen nach dem erfindungsgemäßen Verfahren in wesentlich kürzeren Zeiten und mit besseren Ausbeuten als in dem literaturbekannten Verfahren vorgenommen werden kann. N-Carboxymethylen-4-chlor-anthranilsäure wird unter basischen Bedingungen mit einem Dialkylsulfat, Dialkylcarbonat oder einem Alkylhalogenid, vorteilhaft einem Dialkylsulfat, bevorzugt mit Dimethyl- oder Diethylsulfat umgesetzt. Es werden 1,5 bis 30 Mol, vorteilhaft 1,8 bis 15 Mol, bevorzugt 2 bis 8 mol Dialkylsulfat, Dialkylcarbonat oder Alkylhalogenid pro Mol N-Carboxymethylen-4-chlor-anthranilsäure eingesetzt.

Als Lösungsmittel dient bevorzugt Wasser oder ein polares aprotisches Lösungsmittel, wie z.B. N,N-Dimethylformamid, N,N-Dimethylacetamid, N,N-Diethylacetamid, N-Methylpyrrolidon, Dimethylsulfoxid, Dimethylsulfon, Diphenylsulfon, Sulfolan, Tetramethylharnstoff, Tetra-n-butylharnstoff, 1,3-Dimethylindazolidin-2-on oder Mischungen derselben.

Im allgemeinen ist der Zusatz einer Base günstig. Hierfür können organische basische Verbindungen, z.B. Amine oder anorganische basische Salze, z.B. ein Alkali- oder Erdalkalimetallcarbonat oder -hydroxid eingesetzt werden, vorteilhaft ein Alkalimetallhydroxid oder -carbonat, bevorzugt NaOH, KOH oder Kaliumcarbonat oder Gemische dieser Komponenten. Die Base wird in Mengen von 1 bis 20 Mol, vorteilhaft 2 bis 8 Mol, bevorzugt 2-4 Mol pro Mol Dicarbonsäure eingesetzt.

Die Reaktionstemperaturen hält man zwischen 40 und 150°C, vorteilhaft zwischen 50 und 130°C, bevorzugt zwischen 70 und 120°C.

Die Reaktionszeiten betragen 15 min bis 6 h, vorteilhaft 30 min bis 5 h, bevorzugt 1 bis 4 h.

Die Herstellung der N-Carbalkoxymethylen-4-chlor-anthranilsäureester ist auch in einer Eintopfreaktion möglich. Hierbei werden Ausbeuten größer 80 % (Beispiel 5: 84 %) erhalten.

Bei Durchführung des Verfahrens in 2 Schritten liegen die Ausbeuten bei ca. 85 % (Beispiele 2 und 3).

Es war besonders überraschend, daß das erfindungsgemäße Verfahren mit derart guten Ausbeuten abläuft, da mit dem zweiten Chloratom eine weitere reaktionsfähige Gruppe im Molekül vorliegt und in der EP 71 935 ausdrücklich darauf hingewiesen wird, daß dies zu Reaktionsgemischen führt, die mehrfach substituierte Nebenprodukte enthalten, welche dann chromatographisch abgetrennt werden müssen (Seite 19, Zeilen 27 bis 37).

Die beschriebenen Verfahrensschritte können unter Unter-, Über- oder Normaldruck durchgeführt werden.

Die folgenden Beispiele erläutern die Erfindung, ohne sie zu beschränken.

### Beispiele für die Herstellung von N-Carboxymethylen-4-chlor-anthranilsäure:

### Beispiel 1:

47,8 g (0.25 mol) 2,4-Dichlorbenzoesäure werden in 500 g Dimethylacetamid gelöst, 26,3 g (0,35 mol) Glycin und 1,7 g (7,5 mmol) basisches Kupfercarbonat zugesetzt und auf 80°C erwärmt. Nach Zugabe von 24 g (0,6 mol) NaOH und 22,5 g (0,16 mol) K₂CO₃ wird 5 h auf 140°C erhitzt. Man kühlt auf Raumtemperatur, verdünnt mit 200 ml Wasser, stellt mit Salzsäure auf pH 2 und filtriert das kristalline Produkt ab. Man erhält 43,9 g (0,19 mol, 77 %) N-Carboxymethylen-4-chlor-anthranilsäure.

Bei Verwendung von N-Methylpyrrolidon anstatt Dimethylacetamid als Lösungsmittel erhält man im wesentlichen analoge Ergebnisse.

### Beispiel 2:

In 100 ml Wasser werden 20 g (0,5 mol) NaOH gelöst, 38,2 g (0,2 mol) 2,4-Dichlorbenzoesäure, 22,5 g (0,3 mol) Glycin und 1,4 g (6 mmol) basisches Kupfercarbonat zugegeben. Man erwärmt auf 80°C und versetzt mit 28 g (0,2 mol) Kaliumcarbonat. Es wird für 3 h auf 100°C erhitzt. Man saugt heiß ab, gibt das Filtrat auf 100 ml Wasser und stellt mit Salzsäure auf pH 1-2 ein. Hierbei fällt das Produkt aus, das abgesaugt und getrocknet wird. Man erhält 41 g (0,18 mol; 89 %) N-Carboxymethylen-4-chlor-anthranilsäure.

### Beispiele für die Herstellung von N-Carbalkoxymethylen-4-chloranthranilsäurealkylestern:

### Beispiel 3:

17 g (66 mmol) N-Carboxymethylen-4-chlor-anthranilsäure werden in 175 g N,N-Dimethylformamid gelöst und mit 22,5 g (162 mmol) Kaliumcarbonat versetzt. Bei 80°C werden 56 g (444 mmol) Dimethylsulfat innerhalb 2 h in 3 Portionen zugetropft und 30 min nachgerührt. Der Dimethylsulfatüberschuß wird durch Ammoniakzugabe vernichtet. Das Produkt wird durch Kühlung ausgefällt,abfiltriert und mit Wasser gewaschen. Man isoliert 16,1 g (62,5 mmol; 95 %) N-Carbmethoxymethylen-4-chlor-anthranilsäuremethylester.

### Beispiel 4:

50 g (0,22 mol) N-Carboxymethylen-4-chlor-anthranilsäure, gelöst in 350 ml N,N-Dimethylformamid, werden vorgelegt,mit 60,8g (0,44 mol) Kaliumcarbonat versetzt und auf 80°C geheizt, 121,8 g (0,79 mol) Diethylsulfat werden innerhalb 1,5 h zugetropft, 45 min nachgerührt und innerhalb 75 min weitere 60,9 g (0,4 mol) Diethylsulfat zugetropft. Durch Zugabe von Ammoniak wird der Diethylsulfat-Überschuß zerstört. Nach Filtration der heißen Reaktionsmischung wird das Filtrat auf 185 g Wasser gegeben und durch Kühlung auf 5°C das Produkt ausgefällt. Man erhält 32,6 g (0,114 mol; 52 %) N-Carbethoxymethylen-4-chlor-anthranilsäureethylester.

### Beispiel 5 (Eintopfreaktion):

47,8 g (0,25 mol) 2,4-Dichlorbenzoesäure, 22,5 g (0,3 mol) Glycin,1,5 g (6,8 mmol) basisches Kupfercarbonat und 22 g (0,55 mol) NaOH werden in 300 g N,N-Dimethylacetamid vorgelegt und auf 80°C geheizt. Man versetzt mit 20,8 g (0,15 mol) Kaliumcarbonat und erwärmt auf 100°C. Nach 3,5 h bei 100°C wird die Temperatur auf 110°C gesteigert und 3,5 h bei 110°C gerührt, wobei 100 g N-N-Dimethylacetamid zugegeben werden. Nach Zugabe von weiteren 100 g N-N-Dimethylacetamid erhitzt man 2,5 h auf 120°C und 2,5 h auf 140-150°C. Nach Kühlen auf 80°C werden innerhalb von 2 h 139,8 g (0,9 mol) Diethylsulfat zugetropft, 45 min bei 80°C nachgerührt und mittels Ammoniakzugabe überschüssiges Diethylsulfat vernichtet. Der Reaktionsmischung werden 350 g Wasser zugegeben und das Produkt mit Xylol extrahiert. Man erhält 352 g Xylol-Extrakt mit einem Produktgehalt von ca. 0,17 g/g, dies entspricht 59,8 g (84 %) N-Carbethoxymethylen-4-chloranthranilsäureethylester.

## Patentansprüche

1. Verfahren zur Herstellung von N-Carboxymethylen-4-chlor-anthranilsäure und deren Dialkylestern, dadurch gekennzeichnet, daß man 2,4-Dichlorbenzoesäure in Gegenwart einer Base, eines Lösungsmittels und eines Katalysators aus Kupfer oder Kupferverbindungen mit Glycin zu N-Carboxymethylen-4-chlor-anthranilsäure umsetzt und gegebenenfalls diese in Gegenwart einer Base und eines Lösungsmittels mit einem Dialkylsulfat, Dialkylcarbonat oder Alkylhalogenid zum Dialkylester umsetzt.

2. Verfahren nach Anspruch 1, dadurch gekennzeichnet, daß man pro Mol 2,4-Dichlorbenzoesäure 0,5 bis 10 Mol, insbesondere 0,8 bis 5 Mol, bevorzugt 1 bis 2 Mol Glycin einsetzt.

3. Verfahren nach Anspruch 1 oder 2, dadurch gekennzeichnet, daß man für die Umsetzung von 2,4-Dichlorbenzoesäure mit Glycin ein dipolar aprotisches Lösungsmittel, ein protisch organisches Lösungsmittel oder Wasser einsetzt.

4. Verfahren nach mindestens einem der Ansprüche 1 bis 3, dadurch gekennzeichnet, daß als Lösungsmittel für die Umsetzung mit Glycin N,N-Dimethylacetamid, N,N-Diethylacetamid, N,N-Dimethylformamid, N-Methylpyrrolidon, Dimethylsulfoxid, Dimethylsulfon, Diphenylsulfon, Sulfolan, Tetramethylharnstoff, Tetra-n-butylharnstoff, 1,3-Dimethylindazolidin-2-on, Ethylenglycol, Glyzerin oder Wasser, insbesondere Dimethylacetamid, N-Methylpyrrolidon oder Wasser, eingesetzt werden.

5. Verfahren nach mindestens einem der Ansprüche 1 bis 4, dadurch gekennzeichnet, daß die Konzentration der 2,4-Dichlorbenzoesäure im Lösungsmittel zwischen 1 und 90 Gew.-%, insbesondere 3 und 80 Gew.-%, bevorzugt 5 bis 70 Gew.-% beträgt.

6. Verfahren nach mindestens einem der Ansprüche 1 bis 5, dadurch gekennzeichnet, daß als Katalysator für die Umsetzung der 2,4-Dichlorbenzoesäure mit Glycin, Kupfercarbonat oder Kupferiodid eingesetzt wird.

7. Verfahren nach mindestens einem der Ansprüche 1 bis 6, dadurch gekennzeichnet, daß 0,1 bis 20 mol-%, insbesondere 0,2 bis 10 mol-%, bevorzugt 0,5 bis 5 mol-% des Katalysators, bezogen auf 2,4-Dichlorbenzoesäure, eingesetzt werden.

8. Verfahren nach mindestens einem der Ansprüche 1 bis 7, dadurch gekennzeichnet, daß für die Umsetzung von 2,4-Dichlorbenzoesäure mit Glycin als Base Alkali- oder Erdalkalicarbonate, -hydrogencarbonate, -hydroxide, -oxide, -phosphate, -hydrogenphosphate oder -dihydrogenphosphate, insbesondere Kaliumcarbonat und/oder Natriumhydroxid und/oder Kaliumhydroxid eingesetzt werden.

9. Verfahren nach mindestens einem der Ansprüche 1 bis 8, dadurch gekennzeichnet, daß die Basenmenge für die Umsetzung der 2,4-Dichlorbenzoesäure mit Glycin 1 bis 10 Mol, insbesondere 1,2 bis 6 Mol, bevorzugt 1,5 bis 4 Mol bezogen auf 1 Mol 2,4-Dichlorbenzoesäure beträgt.

10. Verfahren nach mindestens einem der Ansprüche 1 bis 9, dadurch gekennzeichnet, daß für die Umsetzung von 2,4-Dichlorbenzoesäure mit Glycin die Reaktionstemperatur 40 bis 200°C, insbesondere 50 bis 160°C, bevorzugt 60 bis 150°C beträgt.

11. Verfahren nach mindestens einem der Ansprüche 1 bis 10, dadurch gekennzeichnet, daß für die Umsetzung von 2,4-Dichlorbenzoesäure die Reaktionszeiten zwischen 30 min und 20 h, insbesondere zwischen 3 h und 10 h, bevorzugt 2 h bis 8 h betragen.

12. Verfahren nach mindestens einem der Ansprüche 1 bis 11, dadurch gekennzeichnet, daß für die Veresterung 1,5 bis 30 Mol, insbesondere 1,8 bis 15 Mol, bevorzugt 2 bis 8 Mol Dialkylsulfat, Dialkylcarbonat oder Alkylhalogenid pro Mol N-Carboxymethylen-4-chlor-anthranilsäure eingesetzt werden.

13. Verfahren nach mindestens einem der Ansprüche 1 bis 12, dadurch gekennzeichnet, daß die Veresterung mit einem Dialkylsulfat, insbesondere Dimethylsulfat oder Diethylsulfat durchgeführt wird.

14. Verfahren nach mindestens einem der Ansprüche 1 bis 13, dadurch gekennzeichnet, daß als Lösungsmittel für die Veresterung Wasser oder ein polar aprotisches Lösungsmittel, insbesondere N,N-Dimethylformamid, N,N-Dimethylacetamid, N,N-Diethylacetamid, N-Methylpyrrolidon, Dimethylsulfoxid, Dimethylsulfon, Diphenylsulfon, Sulfolan, Tetramethylharnstoff, Tetra-n-butylharnstoff oder 1,3-Dimethylindazolidin-2-on, bevorzugt Wasser, Dimethylformamid oder Dimethylacetamid eingesetzt wird.

15. Verfahren nach mindestens einem der Ansprüche 1 bis 14, dadurch gekennzeichnet, daß als Base für die Veresterung Amine oder Alkali- oder Erdalkalimetallcarbonate oder -hydroxide, insbesondere Natriumhydroxid, Kaliumhydroxid und/oder Kaliumcarbonat eingesetzt werden.

16. Verfahren nach mindestens einem der Ansprüche 1 bis 15, dadurch gekennzeichnet, daß für die Veresterung 1 bis 20 Mol, insbesondere 1,5 bis 8 Mol, bevorzugt 2 bis 4 Mol Base pro Mol Dicarbonsäure eingesetzt werden.

17. Verfahren nach mindestens einem der Ansprüche 1 bis 16, dadurch gekennzeichnet, daß die Reaktionstemperatur für die Veresterung zwischen 40 und 150°C, insbesondere zwischen 50 und 130°C, bevorzugt 70 und 120°C beträgt.

18. Verfahren nach mindestens einem der Ansprüche 1 bis 17, dadurch gekennzeichnet, daß die Reaktionszeiten für die Veresterung zwischen 15 min und 6 h, insbesondere 30 min bis 5 h, bevorzugt 1 bis 4 h betragen.

19. Verfahren nach mindestens einem der Ansprüche 1 bis 18, dadurch gekennzeichnet, daß die Umsetzung der 2,4-Dichlorbenzoesäure mit Glycin und die anschließende Veresterung als Eintopfreaktion durchgeführt werden.

## Claims

1. A process for preparing N-carboxymethylene-4-chloroanthranilic acid and its dialkyl esters, which comprises reacting 2,4-dichlorobenzoic acid in the presence of a base, of a solvent and of a catalyst comprising copper or copper compounds with glycine to give N-carboxymethylene-4-chloroanthranilic acid and, where appropriate, reacting the latter in the presence of a base and of a solvent with a dialkyl sulfate, dialkyl carbonate or alkyl halide to give the dialkyl ester.

2. The process as claimed in claim 1, wherein 0.5 to 10 mol, in particular 0.8 to 5 mol, preferably 1 to 2 mol, of glycine are employed per mole of 2,4-dichlorobenzoic acid.

3. The process as claimed in claim 1 or 2, wherein a dipolar aprotic solvent, a protic organic solvent or water is employed for the reaction of 2,4-dichlorobenzoic acid with glycine.

4. The process as claimed in at least one of claims 1 to 3, wherein the solvent employed for the reaction with glycine is N,N-dimethylacetamide, N,N-diethylacetamide, N,N-dimethylformamide, N-methylpyrrolidone, dimethyl sulfoxide, dimethyl sulfone, diphenyl sulfone, sulfolane, tetramethylurea, tetra-n-butylurea, 1,3-dimethyl-2-indazolidinone, ethylene glycol, glycerol or water, especially dimethylacetamide, N-methylpyrrolidone or water.

5. The process as claimed in at least one of claims 1 to 4, wherein the concentration of 2,4-dichlorobenzoic acid in the solvent is between 1 and 90% by weight, in particular 3 and 80% by weight, preferably 5 to 70% by weight.

6. The process as claimed in at least one of claims 1 to 5, wherein the catalyst employed for the reaction of 2,4-dichlorobenzoic acid with glycine is copper carbonate or copper iodide.

7. The process as claimed in at least one of claims 1 to 6, wherein 0.1 to 20 mol%, in particular 0.2 to 10 mol%, preferably 0.5 to 5 mol%, of the catalyst are employed, based on 2,4-dichlorobenzoic acid.

8. The process as claimed in at least one of claims 1 to 7, wherein alkali metal or alkaline earth metal carbonates, bicarbonates, hydroxides, oxides, phosphates, hydrogen phosphates or dihydrogen phosphates, in particular potassium carbonate and/or sodium hydroxide and/or potassium hydroxide, are employed as base for the reaction of 2,4-dichlorobenzoic acid with glycine.

9. The process as claimed in at least one of claims 1 to 8, wherein the amount of base for the reaction of 2,4-dichlorobenzoic acid with glycine is 1 to 10 mol, in particular 1.2 to 6 mol, preferably 1.5 to 4 mol, based on 1 mol of 2,4-dichlorobenzoic acid.

10. The process as claimed in at least one of claims 1 to 9, wherein the reaction temperature for the reaction of 2,4-dichlorobenzoic acid with glycine is 40 to 200°C, in particular 50 to 160°C, preferably 60 to 150°C.

11. The process as claimed in at least one of claims 1 to 10, wherein the reaction times for the reaction of 2,4-dichlorobenzoic acid are between 30 min and 20 h, in particular between 3 h and 10 h, preferably 2 h to 8 h.

12. The process as claimed in at least one of claims 1 to 11, wherein 1.5 to 30 mol, in particular 1.8 to 15 mol, preferably 2 to 8 mol, of dialkyl sulfate, dialkyl carbonate or alkyl halide are employed per mole of N-carboxymethylene-4-chloroanthranilic acid for the esterification.

13. The process as claimed in at least one of claims 1 to 12, wherein the esterification is carried out with a dialkyl sulfate, in particular dimethyl sulfate or diethyl sulfate.

14. The process as claimed in at least one of claims 1 to 13, wherein the solvent employed for the esterification is water or a polar aprotic solvent, in particular N,N-dimethylformamide, N,N-dimethylacetamide, N,N-diethylacetamide, N-methylpyrrolidone, dimethyl sulfoxide, dimethyl sulfone, diphenyl sulfone, sulfolane, tetramethylurea, tetra-n-butylurea or 1,3-dimethyl-2-indazolidinone, preferably water, dimethylformamide or dimethylacetamide.

15. The process as claimed in at least one of claims 1 to 14, wherein amines or alkali metal or alkaline earth metal carbonates or hydroxides, in particular sodium hydroxide, potassium hydroxide and/or potassium carbonate, are employed as base for the esterification.

16. The process as claimed in at least one of claims 1 to 15, wherein 1 to 20 mol, in particular 1.5 to 8 mol, preferably 2 to 4 mol, of base are employed per mole of dicarboxylic acid for the esterification.

17. The process as claimed in at least one of claims 1 to 16, wherein the reaction temperature for the esterification is between 40 and 150°C, in particular between 50 and 130°C, preferably 70 and 120°C.

18. The process as claimed in at least one of claims 1 to 17, wherein the reaction times for the esterification are between 15 min and 6 h, in particular 30 min to 5 h, preferably 1 to 4 h.

19. The process as claimed in at least one of claims 1 to 18, wherein the reaction of 2,4-dichlorobenzoic acid with glycine and the subsequent esterification are carried out as a one-pot reaction.

## Revendications

1. Procédé de préparation d'acide N-carboxyméthylène-4-chlor-anthranilique et de ses esters dialkyliques, caractérisé en ce qu'on fait réagir l'acide 2,4-dichlorobenzoïque en présence d'une base, d'un solvant et d'un catalyseur à base de cuivre ou de composés de cuivre avec la glycine pour obtenir l'acide N-carboxyméthylène-4-chlor-anthranilique et on fait éventuellement réagir celui-ci en présence d'une base et d'un solvant avec un sulfate de dialkyle, un carbonate de dialkyle ou un halogénure d'alkyle pour obtenir l'ester dialkylique.

2. Procédé selon la revendication 1, caractérisé en ce qu'on utilise de 0,5 à 10 moles, en particulier de 0,8 à 5 moles, de préférence de 1 à 2 moles de glycine par mole d'acide 2,4-dichlorobenzoïque.

3. Procédé selon la revendication 1 ou 2, caractérisé en ce qu'on utilise pour la réaction de l'acide 2,4-dichlorobenzoïque avec la glycine un solvant aprotique dipolaire, un solvant organique protique ou de l'eau.

4. Procédé selon au moins l'une des revendications 1 à 3, caractérisé en ce qu'on utilise, en tant que solvant pour la réaction avec la glycine, le N,N-diméthylacétamide, le N,N-diéthylacétamide, le N,N-diméthylformamide, la N-méthylpyrrolidone, le diméthylsulfoxyde, la diméthylsulfone, la diphénylsulfone, la sulfolane, la tétraméthylurée, la tétra-n-butylurée, la 1,3-diméthyl-indazolidin-2-one, l'éthylène glycol, la glycérine ou l'eau, en particulier le diméthylacétamide, la N-méthylpyrrolidone ou l'eau.

5. Procédé selon au moins l'une des revendications 1 à 4, caractérisé en ce que la concentration en acide 2,4-dichlorobenzoïque dans le solvant est comprise entre 1 et 90% massiques, en particulier entre 3 et 80% massiques, de préférence de 5 à 70% massiques.

6. Procédé selon au moins l'une des revendications 1 à 5, caractérisé en ce qu'on utilise le carbonate de cuivre ou l'iodure de cuivre en tant que catalyseur pour la réaction de l'acide 2,4-dichlorobenzoïque avec la glycine.

7. Procédé selon au moins l'une des revendications 1 à 6, caractérisé en ce qu'on utilise de 0,1 à 20% molaires, de préférence de 0,5 à 5% molaires du catalyseur par rapport à l'acide 2,4-dichlorobenzoïque.

8. Procédé selon au moins l'une des revendications 1 à 7, caractérisé en ce qu'on emploie pour la réaction de l'acide 2,4-dichlorobenzoïque avec la glycine en tant que base des dihydrogénophosphates, des hydrogénophosphates, des phosphates, des oxydes, des hydroxydes, des bicarbonates ou des carbonates alcalins ou de métaux alcalino-terreux, en particulier le carbonate de potassium et/ou l'hydroxyde de sodium et/ou l'hydroxyde de potassium.

9. Procédé selon au moins l'une des revendications 1 à 8, caractérisé en ce que la quantité de base pour la réaction de l'acide 2,4-dichlorobenzoïque avec la glycine va de 1 à 10 moles, en particulier de 1,2 à 6 moles, de préférence de 1,5 à 4 moles, par rapport à 1 mole d'acide 2,4-dichlorobenzoïque.

10. Procédé selon au moins l'une des revendications 1 à 9, caractérisé en ce que, pour la réaction de l'acide 2,4-dichlorobenzoïque avec la glycine, la température réactionnelle est de 40 à 200°C, en particulier de 50 à 160°C, de préférence de 60 à 150°C.

11. Procédé selon au moins l'une des revendications 1 à 10, caractérisé en ce que, pour la réaction de l'acide 2,4-dichlorobenzoïque avec la glycine, la durée réactionnelle est comprise entre 30 minutes et 20 heures, en particulier entre 3 heures et 10 heures, de préférence de 2 heures à 8 heures.

12. Procédé selon au moins l'une des revendications 1 à 11, caractérisé en ce qu'on emploie pour l'estérification de 1,5 à 30 moles, en particulier de 1,8 à 15 moles, de préférence de 2 à 8 moles de sulfate de dialkyle, de carbonate de dialkyle ou d'halogénure d'alkyle par mole d'acide N-carboxyméthylène-4-chloranthranilique.

13. Procédé selon au moins l'une des revendications 1 à 12, caractérisé en ce qu'on met en oeuvre l'estérification avec un sulfate de dialkyle, en particulier le sulfate de diméthyle ou le sulfate de diéthyle.

14. Procédé selon au moins l'une des revendications 1 à 13, caractérisé en ce qu'on emploie en tant que solvant pour l'estérification l'eau ou un solvant polaire aprotique, en particulier le N,N-diméthylformamide, le N,N-diméthylacétamide, le N,N-diéthylacétamide, la N-méthylpyrrolidone, le diméthylsulfoxyde, la diméthylsulfone, la diphénylsulfone, la sulfolane, la tétraméthylurée, la tétra-n-butylurée, la 1,3-diméthylimdazolidin-2-one, de préférence l'eau, en particulier le diméthylformamide ou le diméthylacétamide.

15. Procédé selon au moins l'une des revendications 1 à 14, caractérisé en ce qu'on emploie en tant que base pour l'estérification des amines ou des hydroxydes ou carbonates alcalins ou de métaux alcalino-terreux, en particulier l'hydroxyde de sodium, l'hydroxyde de potassium et/ou le carbonate de potassium.

16. Procédé selon au moins l'une des revendications 1 à 15, caractérisé en ce que, pour l'estérification, on emploie de 1 à 20 moles, en particulier de 1,5 à 8 moles, de préférence de 2 à 4 moles de base par mole d'acide dicarboxylique.

17. Procédé selon au moins l'une des revendications 1 à 16, caractérisé en ce que la température réactionnelle pour l'estérification est comprise entre 40 et 150°C, en particulier entre 50 et 130°C, de préférence entre 70 et 120°C.

18. Procédé selon au moins l'une des revendications 1 à 17, caractérisé en ce que les durées réactionnelles pour l'estérification sont comprises entre 15 minutes et 6 heures, en particulier de 30 minutes à 5 heures, de préférence de 1 à 4 heures.

19. Procédé selon au moins l'une des revendications 1 à 18, caractérisé en ce qu'on met en oeuvre la réaction de l'acide 2,4-dichlorobenzoïque avec la glycine et l'estérification ultérieure sous forme de réaction à réacteur unique.
